(19) Europäisches Patentamt
European Patent Office
Office européen des brevets

(11) EP 1 437 347 A2

(12) **EUROPÄISCHE PATENTANMELDUNG**

(43) Veröffentlichungstag:
**14.07.2004 Patentblatt 2004/29**

(51) Int Cl.[7]: **C07D 231/12**, A01N 43/56

(21) Anmeldenummer: **03028914.4**

(22) Anmeldetag: **17.12.2003**

(84) Benannte Vertragsstaaten:
**AT BE BG CH CY CZ DE DK EE ES FI FR GB GR HU IE IT LI LU MC NL PT RO SE SI SK TR**
Benannte Erstreckungsstaaten:
**AL LT LV MK**

(30) Priorität: **07.01.2003 DE 10300124**

(71) Anmelder: **Bayer Chemicals AG**
**51368 Leverkusen (DE)**

(72) Erfinder:
- **Ebenbeck, Wolfgang, Dr.**
  **51373 Leverkusen (DE)**
- **Rampf, Florian, Dr.**
  **50733 Köln (DE)**

(54) **Verfahren zur Herstellung von Arylvinylhalogeniden und Arylalkinen**

(57) Die Erfindung betrifft ein Verfahren zur Herstellung von Arylvinylhalogeniden und -sulfonaten und Arylalkinen durch Umsetzung von Halogenaromaten oder Arylsulfonaten mit Vinylhalogeniden oder -sulfonaten in Gegenwart von Palladiumkatalysator und Base sowie gegebenenfalls anschließender Eliminierung.

EP 1 437 347 A2

Printed by Jouve, 75001 PARIS (FR)

## Beschreibung

Die Erfindung betrifft ein Verfahren zur Herstellung von Arylvinylhalogeniden und -sulfonaten und Arylalkinen durch Umsetzung von Halogenaromaten oder Arylsulfonaten mit Vinylhalogeniden oder -sulfonaten in Gegenwart von Palladiumkatalysator und Base sowie gegebenenfalls anschließender Eliminierung.

Arylalkine besitzen als Feinchemikalien, Ausgangsprodukte für Polymere und Wirkstoffzwischenprodukte eine hohe industrielle Bedeutung (siehe auch EP-A 571 326 und EP-A 1 219 173).

Die Herstellung von Arylalkinen kann beispielsweise aus Arylaldehyden mit Triphenylphosphan und $C_1$-Bausteinen wie Methylenchlorid oder Tetrabrommethan in Gegenwart starker Basen erfolgen (Chem. Ber., **1982**, *115*, 828 ff.). Weiterhin ist die Umsetzung elektronenreicher Aromaten unter Friedel-Crafts-Bedingungen zu Acetylaromaten mit anschließender Halogenierung und Halogenwasserstoff-Eliminierung bekannt (Chem. Ber. **1965,** *98,* 3554 ff.).

Nachteilig an diesen Verfahren sind die oftmals drastischen Bedingungen und die allenfalls mäßigen Ausbeuten.

Weiterhin ist für die Darstellung von Arylalkinen die Umsetzung von Halogenaromaten mit Trimethylsilylacetylen (Chem. Comm. **2002**, 278), Acetylen (JP-A 2001 294541), Trialkylzinnacetylen (Org. Lett. 2001, 3, 1869) oder Alkinyl-Grignard-Verbindungen (J. Org. Chem., **1997**, 62, 8957-8960) in Gegenwart von Palladium-Katalysatoren bekannt.

In allen genannten Verfahren ist die eingeschränkte Verfügbarkeit der Alkinbausteine in industriell relevanten Mengen und/oder ihre schwierige Handhabung und/oder der unerwünscht hohe Gehalt an Schwermetallen ein erheblicher Nachteil.

Darüber hinaus ist weiterhin die Herstellung von Arylalkinen beispielsweise aus Arylolefinen durch Halogenierung der Doppelbindung und anschließende Eliminierung bekannt. Nachteilig daran ist jedoch die Durchführung von mindestens drei Reaktionsschritten.

Es bestand daher das Bedürfnis, ein Verfahren zu entwickeln, das die Darstellung von Arylalkinen oder besonders geeigneten Vorprodukten davon aus Halogenaromaten oder Arylsulfonaten in effizienter Weise ermöglicht.

Es wurde nun ein Verfahren zur Herstellung von Arylvinylhalogeniden oder -sulfonaten sowie Arylalkinen gefunden, das dadurch gekennzeichnet ist, dass in einem Schritt

**A)**

- Verbindungen der Formel (I),

$$Ar\text{-}[X]_n \qquad (I),$$

  in der

  n für eins oder zwei steht und

  Ar für einen substituierten oder unsubstituierten aromatischen oder für einen substituierten oder unsubstituierten polyaromatischen Rest und

  X jeweils unabhängig von n für Chlor, Brom, Iod, ein Sulfonat oder ein Diazoniumsalz steht,

- in Gegenwart von Palladium-Katalysator und
- in Gegenwart mindestens einer Base und
- gegebenenfalls in Gegenwart eines Salzes und
- gegebenenfalls in Gegenwart von Lösungsmittel
- mit Verbindungen der Formel (II),

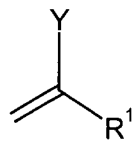

(II)

in der

Y für Fluor, Chlor, Brom, Iod, ($C_1$-$C_{12}$-Alkyl)sulfonyloxy oder ($C_1$-$C_{12}$-Halogenalkyl)sulfonyloxy und

$R^1$ für Wasserstoff, Cyano, $C_1$-$C_{12}$-Alkyl, $C_6$-$C_{15}$-Arylalkyl, $C_5$-$C_{14}$-Aryl, Fluor, Chlor, COO($C_5$-$C_{14}$-Aryl), COO($C_1$-$C_{12}$-Alkyl), CON($C_5$-$C_{14}$-Aryl)$_2$, CON($C_1$-$C_{12}$-Alkyl)$_2$, OCO($C_5$-$C_{14}$-Aryl) oder OCO ($C_1$-$C_{12}$-Alkyl) steht zu Verbindungen der Formel (III) umgesetzt werden,

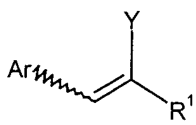

(III)

und gegebenenfalls in einem Schritt

**B)**

- die Verbindungen der Formel (III) durch Umsetzung mit Base zu Verbindungen der Formel (IV)

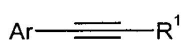

(IV)

umgesetzt werden.

Im Rahmen der Erfindung können alle oben stehenden und im Folgenden aufgeführten, allgemeinen oder in Vorzugsbereichen genannten Restedefinitionen, Parameter und Erläuterungen untereinander, also auch zwischen den jeweiligen Bereichen und Vorzugsbereichen in beliebiger Weise kombiniert werden.

Alkyl bzw. Alkylen, bzw. Alkoxy steht im Rahmen der Erfindung jeweils unabhängig für einen geradkettigen, cyclischen, verzweigten oder unverzweigten Alkyl- bzw. Alkylen- bzw. AlkoxyRest, der gegebenenfalls durch $C_1$-$C_4$-Alkoxy-Reste weiter substituiert sein kann. Gleiches gilt für den Alkylenteil eines Arylalkyl-Restes.

Beispielsweise steht $C_1$-$C_4$-Alkyl für Methyl, Ethyl, n-Propyl, Isopropyl, n-Butyl und tert.-Butyl, $C_1$-$C_8$-Alkyl darüber hinaus beispielsweise für n-Pentyl, Cyclohexyl, n-Hexyl, n-Heptyl, n-Octyl oder iso-Octyl, $C_1$-$C_{12}$-Alkyl, weiter darüber hinaus z.B. für n-Decyl und n-Dodecyl und $C_1$-$C_{20}$ noch weiter darüber hinaus für n-Hexadecyl und n-Octadecyl.

Beispielsweise steht $C_1$-$C_4$-Alkylen für Methylen, 1,1-Ethylen, 1,2-Ethylen, 1,1-Propylen, 1,2-Propylen, 1,3-Propylen, 1,1-Butylen, 1,2-Butylen, 2,3-Butylen und 1,4-Butylen, $C_1$-$C_8$-Alkylen darüber hinaus für 1,5-Pentylen, 1,6-Hexylen, 1,1-Cyclohexylen, 1,4-Cyclohexylen, 1,2-Cyclohexylen und 1,8-Octylen.

Beispielsweise steht $C_1$-$C_4$-Alkoxy für Methoxy, Ethoxy, Isopropoxy, n-Propoxy, n-Butoxy und tert.-Butoxy, $C_1$-$C_8$-Alkoxy darüber hinaus für Cyclohexyloxy.

Die allgemeine Bezeichnung Aryl als Substituent umfasst carbocyclische Reste und heteroaromatische Reste in denen keines, ein, zwei oder drei Gerüstatome pro Cyclus, im gesamten Rest mindestens jedoch ein Gerüstatom Heteroatome sind, die aus der Gruppe Stickstoff, Schwefel oder Sauerstoff ausgewählt sind und die weiterhin einen oder mehrere Substituenten tragen können, die jeweils unabhängig voneinander ausgewählt sind aus der Gruppe Fluor, Nitro, Cyano, Di($C_1$-$C_8$alkyl)-amino, $C_1$-$C_8$-Alkyl, $C_5$-$C_{14}$-Aryl, $C_1$-$C_8$-Fluoralkyl, $C_1$-$C_8$-Fluoralkoxy, $C_1$-$C_8$-Alkoxy, CO($C_1$-$C_8$-Alkyl), COO-($C_1$-$C_8$)-Alkyl und -CON($C_1$-$C_8$-Alkyl)$_2$.

Gleiches gilt für den Arylteil eines Arylalkyl-Restes. $C_6$-$C_{15}$-Arylalkyl steht beispielsweise und bevorzugt für

Benzyl.

Halogenalkyl bzw. Fluoralkyl bedeutet im Rahmen der Erfindung jeweils unabhängig einen geradkettigen, cyclischen, verzweigten oder unverzweigten Alkylrest, der mit einem, mehreren oder vollständig mit Halogenatomen substituiert ist, die unabhängig voneinander ausgewählt sind aus der Gruppe Fluor, Chlor oder Brom bzw. Fluor.

Beispielsweise und bevorzugt steht $C_1$-$C_8$-Halogenalkyl für Trifluormethyl, Trichlormethyl, 2,2,2-Trifluorethyl, Pentafluorethyl und Nonafluorbutyl, $C_1$-$C_8$-Fluoralkyl für Trifluormethyl, 2,2,2-Trifluorethyl, Pentafluorethyl und Nonafluorbutyl.

Geschütztes Formyl bedeutet einen Formyl-Rest, der durch Überführung in ein Aminal, Acetal oder ein gemischtes Aminalacetal geschützt ist, wobei die Aminale, Acetale und gemischten Aminalacetale acyclisch oder cyclisch sein können.

Beispielsweise und bevorzugt steht geschütztes Formyl für einen 1,1-(2,5-Dioxy)-cyclopentylen-Rest.

Im Folgenden werden die Vorzugsbereiche für Verbindungen der Formeln (I) bis (IV) definiert:

Ar steht bevorzugt für carbocyclische aromatische Reste mit 6 bis 24 Gerüstkohlenstoffatomen, für carbocyclische bis- oder trisaromatische Reste mit 6 bis 10 Kohlenstoffatomen pro aromatischen Rest oder für heteroaromatische Reste mit 5 bis 24 Gerüstatomen, in denen keines, ein, zwei oder drei Gerüstatome pro Cyclus, im gesamten Molekül mindestens jedoch ein Gerüstatom Heteroatome sind, die ausgewählt sind aus der Gruppe Stickstoff, Schwefel oder Sauerstoff. Weiterhin können die carbocyclischen aromatischen Reste, die carbocyclischen bis- oder trisaromatischen Reste oder die heteroaromatischen Reste mit bis zu fünf gleichen oder verschiedenen Substituenten pro Cyclus substituiert sein, die ausgewählt sind aus der Gruppe Hydroxy, Chlor, Fluor, Nitro, Cyano, freies oder geschütztes Formyl, $C_1$-$C_{12}$-Alkyl, $C_5$-$C_{14}$-Aryl, $C_6$-$C_{15}$-Arylalkyl, -PO-[($C_1$-$C_8$)-Alkyl]$_2$, -PO-[($C_5$-$C_{14}$)-Aryl]$_2$, -PO-[($C_1$-$C_8$)-Alkyl)($C_5$-$C_{14}$)-Aryl)], Tri($C_1$-$C_8$-alkyl)-siloxyl oder Resten der Formel (Va-f),

A-B-D-E (Va)

A-E (Vb)

A-$SO_2$-E (Vc)

A-B-$SO_2R^3$ (Vd)

A-$SO_3$W (Ve)

A-COW (Vf)

in denen unabhängig voneinander

A fehlt oder für einen $C_1$-$C_8$-Alkylenrest steht und

B fehlt oder für Sauerstoff, Schwefel oder $NR^2$ steht, wobei $R^2$ Wasserstoff, $C_1$-$C_8$-Alkyl, $C_6$-$C_{15}$-Arylalkyl oder $C_5$-$C_{14}$-Aryl bedeutet und

D für eine Carbonyl-Gruppe steht und

E für $R^3$, $OR^3$, $NHR^4$ oder $N(R^4)_2$ steht, wobei $R^3$ für $C_1$-$C_8$-Alkyl, $C_6$-$C_{15}$-Arylalkyl, $C_1$-$C_8$-Halogenalkyl oder

$C_5$-$C_{14}$-Aryl und $R^4$ jeweils unabhängig für $C_1$-$C_8$-Alkyl, $C_6$-$C_{15}$-Arylalkyl oder $C_5$-$C_{14}$-Aryl oder $N(R^4)_2$ zusammen für einen cyclischen Aminorest steht und

W für OH, $NH_2$, oder OM steht, wobei M ein Alkalimetallion, ein halbes Äquivalent eines Erdalkalimetallions, ein Ammoniumion oder ein organisches Ammoniumion bedeuten kann.

**Ar** steht besonders bevorzugt für Phenyl, Naphthyl, Phenanthrenyl, Anthracenyl, Biphenyl, Binaphthyl, Fluorenyl, Pyridinyl, Oxazolyl, Thiophen-yl, Benzofuranyl, Benzothiophenyl, Dibenzofuran-yl, Dibenzothiophen-yl, Furanyl, Indolyl, Pyridazinyl, Pyrazinyl, Pyrimidinyl, Triazolyl und Chinolinyl, wobei die genannten Reste weiterhin mit keinem, einem, zwei oder drei Resten pro Cyclus weiter substituiert sein können, die jeweils unabhängig voneinander ausgewählt sind aus der Gruppe Fluor, Nitro, Cyano, Di($C_1$-$C_4$alkyl)-amino, $C_1$-$C_4$-Alkyl, $C_5$-$C_{10}$-Aryl, $C_1$-$C_8$-Fluoralkyl, $C_1$-$C_8$-Fluoralkoxy, $C_1$-$C_8$-Alkoxy, CO($C_1$-$C_4$-Alkyl), COO-($C_1$-$C_4$)-Alkyl, -CON($C_1$-$C_4$-Alkyl)$_2$.

**Ar** steht ganz besonders bevorzugt für einen Phenyl-Rest, der mit keinem, einem, zwei oder drei Resten weiter substituiert sein kann, die jeweils voneinander unabhängig ausgewählt sind aus der Gruppe Fluor, Cyano, $C_1$-$C_4$-Alkyl, $C_1$-$C_4$-Alkoxy, Trifluormethyl, Trifluormethoxy, Acetyl, COO-($C_1$-$C_4$)-Alkyl und -CON($C_1$-$C_4$-Alkyl)$_2$.

**n** steht bevorzugt für 1.

**X** steht bevorzugt für Chlor, Brom, Iod, Trifluormethansulfonyloxy oder Nonafluorbutansulfonyloxy und besonders bevorzugt für Chlor oder Brom.

Ganz besonders bevorzugt werden als Verbindungen der Formel (I) eingesetzt:
p-Trifluormethylbrombenzol, o-Trifluormethylbrombenzol, m-Trifluormethylbrombenzol, 3,5-Bis-trifluormethylbrombenzol, o-Cyanobrombenzol, p-Brombenzaldehyd und 4-Brom-1,5-dimethyl-3-(trifluoromethyl)-1H-pyrazol.

**Y** steht bevorzugt für Fluor, Chlor, Brom, Iod, Methansulfonyloxy, p-Tolylsulfonyloxy, Trifluormethan-sulfonyloxy oder Nonafluorbutansulfonyloxy und ganz besonders bevorzugt für Fluor, Chlor und Brom, wobei Chlor noch weiter bevorzugt ist.

**$R^1$** steht bevorzugt für Wasserstoff, Cyano, $C_1$-$C_{12}$-Alkyl, $C_5$-$C_{14}$-Aryl, Fluor, Chlor, COO($C_5$-$C_{14}$-Aryl), COO ($C_1$-$C_{12}$-Alkyl), OCO($C_5$-$C_{14}$-Aryl) oder OCO($C_1$-$C_{12}$-Alkyl), besonders bevorzugt für Wasserstoff, Cyano, Fluor, oder Chlor und ganz besonders bevorzugt für Wasserstoff.

Bevorzugte Verbindungen der Formel (II) sind Vinylfluorid, Vinylchlorid, Vinylbromid, 2-Chloracrylnitril, 2-Chloracrylsäuremethylester, 2-Chloracrylsäurebutylester, 1,1-Dichlorethylen, 1,1-Difluorethylen und 4-(1-Chlorethenyl)-1,5-dimethyl-3-(trifluoromethyl)-1H-pyrazol, wobei Vinylchlorid besonders bevorzugt ist.
Schritt A) des erfindungsgemäßen Verfahrens wird in Gegenwart von Palladium-Katalysator durchgeführt.
Als Palladiumkatalysator werden beispielsweise und bevorzugt Palladiumkomplexe eingesetzt.
Palladiumkomplexe können beispielsweise aus Palladium-Verbindungen und geeigneten Liganden in der Reaktionslösung erzeugt werden, oder in Form bereits isolierter Palladium-Komplexe eingesetzt werden.
Für das erfindungsgemäße Verfahren sind als isolierte Palladium-Komplexe beispielsweise Palladium-Komplexe geeignet, die als Liganden Phosphorverbindungen wie z.B. Phosphine, Phosphite, Phosphonite oder Mischungen davon, bevorzugt Phosphine enthalten.
Als Palladium-Komplexe, die als Liganden Phosphorverbindungen enthalten können beispielsweise und bevorzugt solche der Formel (VIa) verwendet werden,

$$[PdL_2An_2] \qquad \text{(VIa)}$$

in der

L für jeweils eine Monophosphorverbindung oder

$L_2$ zusammen für ein Diphosphorverbindung und

An für ein Anion, bevorzugt für Chlorid, Bromid, Iodid, Acetat, Propionat, Allyl oder Cyclopentadienyl steht.

oder solche der Formel (VIb),

$$[PdL_m] \quad \text{(VIb)}$$

in der

m für 2, 3 oder 4 und

in der

L jeweils für eine Monophosphorverbindung oder ein halbes Äquivalents einer Diphosphorverbindung stehen kann.

Monophosphorverbindungen sind beispielsweise und bevorzugt solche der Formel (VIIa),

$$P(G\text{-}R^5)_3 \quad \text{(VIIa)}$$

in der

G jeweils unabhängig voneinander und unabhängig von $R^5$ fehlen oder für Sauerstoff stehen und die Reste $R^5$ jeweils unabhängig voneinander für $C_1$-$C_8$-Alkyl oder unsubstituiertes, ein-, zwei oder dreifach durch $R^6$ substituiertes Phenyl-, Naphthyl- oder Ferrocenyl stehen, wobei

$R^6$ für $C_1$-$C_8$-Alkyl, $C_1$-$C_8$-Alkoxy, Chlor, Fluor-, $N(C_1$-$C_6$-$Alkyl)_2$, $CO_2$-($C_1$-$C_8$-Alkyl), -CON($C_1$-$C_8$-$Alkyl)_2$, Cyano- oder CO($C_1$-$C_8$-Alkyl), steht.

Besonders bevorzugte Monophosphorverbindungen sind solche der Formel (VIIa), in der G fehlt und $R^5$ unabhängig voneinander für $C_1$-$C_8$-Alkyl oder unsubstituiertes, ein-, zwei oder dreifach durch $R^6$ substituiertes Phenyl- oder Naphthyl- oder Ferrocenyl stehen, wobei

$R^6$ für $C_1$-$C_8$-Alkyl, $C_1$-$C_8$-Alkoxy, Chlor oder Fluor steht.

Ganz besonders bevorzugt sind Monophosphorverbindungen sind solche der Formel (VIIa), in der G fehlt und zwei oder drei der Reste $R^5$ unabhängig voneinander für $C_1$-$C_8$-Alkyl und keiner oder ein Rest $R^5$ für unsubstituiertes, ein-, zwei oder dreifach durch $R^6$ substituiertes Phenyl- oder Naphthylsteht, wobei

$R^6$ für $C_1$-$C_8$-Alkyl, $C_1$-$C_8$-Alkoxy, Chlor oder Fluor steht.

Noch weiter bevorzugt sind als Monophosphorverbindungen Triphenylphosphin, Tri-(tert.-butyl)-phosphin, Phenyldi(tert.-butyl)phosphin und Ferrocenyl-di-(tert.-butyl)phosphin.

Diphosphorverbindungen sind beispielsweise und bevorzugt solche Formel (VIIb),

$$(R^7\text{-}G)_2P\text{-}G\text{-}Z\text{-}G\text{-}P(G\text{-}R^7)_2 \quad \text{(VIIb)}$$

in der

G jeweils unabhängig voneinander und unabhängig von $R^7$ und Z fehlt oder für Sauerstoff steht und die Reste $R^7$ unabhängig voneinander für $C_1$-$C_8$-Alkyl oder für unsubstituiertes, ein-, zwei oder dreifach durch $R^8$ substituiertes Phenyl- , Naphthyl- oder Heteroaryl mit 5 bis 12 Gerüstkohlenstoffatomen stehen, wobei

$R^8$ jeweils unabhängig ausgewählt ist aus der Gruppe $C_1$-$C_8$-Alkyl, $C_1$-$C_8$-Alkoxy, Fluor- oder Cyano- und

Z für einen unsubstituierten oder substituierten Rest aus der Gruppe $C_1$-$C_4$-Alkylen, 1,2-Phenylen, 1,3-Phenylen; 1,2-Cyclohexyl,. 1,1'-Ferrocenyl, 1,2-Ferrocenyl, 2,2'-(1,1'-Binaphthyl) und 1,1'-Biphenyl steht.

Bevorzugte Diphosphorverbindungen sind 1,3-Bis(diisopropylphosphino)propan, 1,4-Bis(diisopropylphosphino)butan, 2,2'-Bis(diphenylphosphino)-1,1'-binaphthyl.
Bevorzugt werden Komplexe eingesetzt, die als Liganden Monophosphorverbindungen enthalten.
Bevorzugte isolierte Palladiumkomplexe sind Palladium(II)bis(tri-t-butylphosphin)-dichlorid, Palladium(II)bis-di-tert.-butylphenylphosphindichlorid, Palladium(II)bis-di-tert.-butylferrocenylphosphindichlorid, Palladium(0)tricyclohexylphosphandiallylether-Komplex, Palladium(0)bistricyclohexylphosphan.
Für das erfindungsgemäße Verfahren sind als Palladiumkatalysatoren Palladiumkomplexe bevorzugt, die aus Palladium-Verbindungen und Liganden in der Reaktionslösung erzeugt werden.
Als Palladiumverbindungen können beispielsweise und bevorzugt eingesetzt werden $Pd_2$(dibenzylidenaceton)$_3$ oder Allylpalladiumchlorid oder -bromid oder solche der Formel (VIIIa),

$$Pd(Y^1)_2 \qquad \text{(VIIIa)}$$

in der

$Y^1$ für ein Anion, bevorzugt für Chlorid, Bromid, Acetat, Propionat, Nitrat, Methansulfonat, Trifluormethansulfonat, Acetylacetonat, Allyl oder Cyclopentadienyl steht,

oder Palladiumverbindungen der Formel (VIIIb),

$$Pd(Y^2)_2L_2 \qquad \text{(VIIIb)}$$

in der

$Y^2$ für ein Anion, bevorzugt Chlorid, Bromid, Acetat, Methansulfonat, Nonafluorbutansulfonat oder Trifluormethansulfonat Tetrafluoroborat oder Hexafluorophosphat steht und

L jeweils für ein Nitril, bevorzugt Acetonitril, Benzonitril oder Benzylnitril, oder ein Olefin, bevorzugt Cyclohexen oder Cycloocten, steht, oder

$L_2$ zusammen für ein Diolefin, bevorzugt Norbomadien oder 1,5-Cyclooctadien steht, oder Palladiumverbindungen der Formel (VIIIc),

$$M_2[Pd(Y^3)_4] \qquad \text{(VIIIc)}$$

wobei

$Y^3$ für ein Halogenid, bevorzugt Chlorid oder Bromid steht und

M für Lithium, Natrium, Kalium, Ammonium oder organisches Ammonium steht.

Bevorzugt sind als Palladiumverbindungen Palladium(II)acetat, Palladium(II)chlorid, Palladium(II) bromid, Palladium(II)propionat, Palladium(II)acetylacetonat, Lithium-, Natriumoder Kaliumtetrachloropalladat, Palladium(II) chlorid-bis-benzonitril, Palladium(II)chlorid-bisacetonitril.
Bevorzugt werden für die Erzeugung von Palladiumkomplexen in der Reaktionslösung als Liganden Phosphorverbindungen der Formeln (VIIa) und (VIIb) verwendet, wobei Monophosphorverbindungen der Formel (VIIa) noch weiter bevorzugt sind. Die genannten Vorzugsbereiche gelten dabei in gleicher Weise.
Das molare Verhältnis von Phosphor zu Palladium in der Reaktionsmischung kann beispielsweise 1:1 1 bis 10:1 1 betragen, 2:1 1 bis 5:1 1 ist bevorzugt, besonders bevorzugt ist 3:1 1 bis 4:1.
Für den erfindungsgemäßen Schritt A) kann das molare Verhältnis von auszutauschendem X in Verbindungen der Formel (I) zu Palladium beispielsweise 10 bis 20000 betragen, bevorzugt ist ein Verhältnis von 50 bis 5000, ganz besonders bevorzugt 100 bis 2000.
Das erfindungsgemäße Verfahren wird in Gegenwart mindestens einer, bevorzugt genau einer Base durchgeführt.

Als Basen sind beispielsweise ionische Basen, Amine oder N-heteroaromatische Verbindungen geeignet.
Bevorzugte Amine sind beispielsweise und bevorzugt solche der Formel (IX),

$$NR^9R^{10}R^{11} \qquad (IX)$$

in der

$R^9$, $R^{10}$ und $R^{11}$ jeweils unabhängig voneinander für $C_1$-$C_{12}$-Alkyl, $C_5$ bis $C_{14}$-Aryl oder $C_6$-$C_{15}$-Arylalkyl stehen oder jeweils zwei oder drei der Reste $R^9$, $R^{10}$ und $R^{11}$ mit dem Stickstoffatom einen mono-, bi- oder tricyclischen Heterocyclus mit 4 bis 8 Kohlenstoffatomen pro Cyclus bilden können.

Ionische Basen im Sinne der Erfindung sind beispielsweise Alkali- und Erdalkalimetallcarboxylate wie beispielsweise Acetate, Propionate, Benzoate, Alkali- und Erdalkalimetall-Alkoholate,Amide, -Hydride, Alkali- und Erdalkalimetall-Carbonate, -Hydrogencarbonate, -Phosphate, - Hydrogenphosphate, -Hydroxide. Alkalimetalle sind bevorzugt Lithium, Natrium, Kalium und Cäsium, Erdalkalimetalle bevorzugt Calcium, Magnesium und Barium.
Bevorzugte N-heteroaromatische Verbindungen sind beispielsweise Pyridine, wie Pyridin, 2,6-Dimethyl-pyridin, 2-,3- oder 4-N,N-Dimethylaminopyridin oder 2-,3- oder 4-Picolin sowie Chinoline, wie Chinolin oder 2-Methylchinolin.
Besonders bevorzugt werden für das erfindungsgemäße Verfahren sterisch anspruchsvolle Stickstoffbasen wie Ethyldiisopropylamin, Triisopropylamin, Diisopropylanilin, Triisobutylamin, Ethyldiisobutylamin, Dicyclohexylmethylamin, Dicyclohexyethylamin, Cyclohexyldiethylamin, Cyclohexyldimethylamin und 2,6-Bis-diisopropylpyridin eingesetzt, wobei Dicyclohexylmethylamin, Dicyclohexyethylamin und Cyclohexyldimethylamin bevorzugt sind.
Die Menge der eingesetzten Base kann z.B. das 0,8- bis 200-fache, bevorzugt das 1- bis 3-fache und noch weiter bevorzugt das 1,0- bis 1,2-fache, bezogen auf die molare Menge an auszutauschendem X in Verbindungen der Formel (I) sein.
Das erfindungsgemäße Verfahren wird gegebenenfalls in Gegenwart eines Salzes, durchgeführt.
Als Salze sind für das erfindungsgemäße Verfahren beispielsweise und bevorzugt Salze der Formel (X) geeignet,

$$(Kation^+)(Anion^-) \qquad (X)$$

in der

(Kation⁺) für substituierte Ammonium, Phosphonium oder Arsonium-Kationen oder Alkalimetallionen und

(Anion⁻) für das Anion einer organischen oder anorganischen Säure steht.

Bevorzugt steht (Kation⁺) für Kationen der Formel (XI),

$$[Pnyc(C_1\text{-}C_{12}\text{-Alkyl})_p(C_6\text{-}C_{15}\text{-Arylalkyl})_q(C_5\text{-}C_{14}\text{-Aryl})_r]^+ \qquad (XI)$$

in der
Pnyc für Stickstoff, Phosphor oder Arsen, bevorzugt für Stickstoff steht und
(p+q+r) = 4 ergibt.
Besonders bevorzugt steht (Kation⁺) für Tetrabutylammonium, Tetraphenylammonium, Tetraphenylphosphonium, Tetrabutylphosphonium.
Bevorzugt steht (Anion⁻) für Fluorid, Chlorid, Bromid, Iodid, Cyanat, Thiocyanat, Acetat, Hydroxid, Nitrat, Hydrogensulfat, Tetrafluoroborat, Hexafluorophosphat, Tosylat, und Triflat, besonders bevorzugt für Chlorid, Bromid, Iodid.
Ganz besonders bevorzugte Salze sind Tetrabutylammoniumchlorid, Tetrabutylammoniumbromid, Tetraphenylammoniumbromid, Tetrabutylphosphoniumchlorid, Tetrabutylphosphoniumbromid, Tetraphenylphosphoniumchlorid und Tetraphenylphosphoniumbromid oder Mischungen davon.
Noch weiter bevorzugt ist Tetrabutylammoniumbromid.
Die Salze können beispielsweise in Mengen von 0,01 bis 100 mol-% bezogen auf die die theoretische Ausbeute limitierende Verbindung (Verbindung der Formel (I) oder Verbindung der Formel (II)) eingesetzt werden, bevorzugt in Mengen von 0,1 bis 15 mol-%, besonders bevorzugt in Mengen von 0,5 bis 5 mol-%.

Größere Mengen wie auch Salzschmelzen sind möglich, aber unwirtschaftlich.

Die Menge des eingesetzten Verbindung der Formel (II) kann beispielsweise das 0,2 bis 200-fache (bei Verwendung als Lösungsmittel) bezogen auf die molare Menge an auszutauschendem X in der Verbindung der Formel (I) sein, das 0,5- bis 30-fache ist bevorzugt, das 0,8- bis 5-fache ist besonders bevorzugt.

Werden Verbindungen der Formel (I) verwendet, die freie Säuregruppen wie z.B. Sulfonsäureoder Carbonsäuregruppen tragen, so ist die Menge der eingesetzten Base entsprechend zu erhöhen.

Gegebenenfalls wird Schritt A) in Gegenwart von Lösungsmittel, bevorzugt in Gegenwart von aprotischem Lösungsmittel, besonders bevorzugt in Gegenwart von polar aprotischem Lösungsmittel durchgeführt.

Aprotisch bedeutet dabei, dass das Lösungsmittel keine Protonen enthält, die bezogen auf eine wässrige Vergleichsskala bei 25°C einen pKs-Wert von weniger als 20 aufweisen.

Polar bedeutet dabei, dass das Lösungsmittel bei 25°C eine Dielektrizitätskonstante $\varepsilon$ von mindestens 4 aufweist.

Bevorzugte aprotische Lösungsmittel sind:

Ether wie z.B. Dioxan, THF, 1,2-Dimethoxyethan, Diethylenglykoldimethylether, Tetraethylenglykoldimethylether; amidische Lösungsmittel wie beispielsweise Dimethylformamid, N-Methylpyrrolidon, N-Methylcaprolactam oder Dimethylacetamid; Nitrile wie beispielsweise Acetonitril, Benzonitril und Benzylnitril; Ketone wie z.B. Dimethylketon, Diethylketon und Methyl-tert.-butylketon; Sulfoxide wie zum Beispiel Dimethylsulfoxid sowie Sulfone wie zum Beispiel Tetramethylensulfon oder Mischungen solcher Lösungsmittel.

Die Menge des gegebenenfalls eingesetzten Lösungsmittels kann beispielsweise 50 ml bis 5000 ml bevorzugt 100 bis 500 ml pro Mol der Verbindung der Formel (I) sein.

Die Reaktionstemperatur in Schritt A) kann zum Beispiel 20°C bis 200°C, bevorzugt 80 bis 150°C und besonders bevorzugt 100°C bis 140°C betragen.

Die Reaktion gemäß Schritt A) kann beispielsweise bei 0,2 bis 100 bar durchgeführt werden, bevorzugt ist Umgebungsdruck oder derjenige Druck, der sich in einem geschlossenen Gefäß bei Reaktionstemperatur bildet.

Die Reaktionsdauer in Schritt A) kann beispielsweise 0,2 h bis 72 Stunden betragen, 1 bis 20 h sind bevorzugt.

Die Reaktion wird bevorzugt unter Schutzgasatmosphäre unter weitgehendem Ausschluss von Sauerstoff und Feuchtigkeit durchgeführt. Als Schutzgase kommen beispielsweise Stickstoff und Edelgase wie beispielsweise Argon oder Mischungen solcher Gase in Frage.

In einer bevorzugten Ausführungsform des erfindungsgemäßen Schrittes A) legt man in einem druckfesten Reaktionsgefäß die Verbindung der Formel (I) zusammen mit der Verbindung der Formel (II), der Base, gegebenenfalls dem Salz, dem Liganden, und der Palladiumverbindung in einem Reaktionsgefäß unter Schutzgas vor und erwärmt den Ansatz unter Rühren auf die Reaktionstemperatur. Nach beendeter Reaktion gießt man die Mischung auf Wasser. Feste Produkte fallen dann aus und können abgesaugt und z.B. mit Wasser gewaschen werden. Flüssige Produkte können mit einem organischen, mit Wasser nicht oder wenig mischbaren Lösungsmittel extrahiert und beispielsweise destillativ aufgearbeitet werden.

Wird eine gasförmige Verbindung der Formel (II) eingesetzt, so wird dieses vorzugsweise als letzte Reaktionskomponente dem Ansatz hinzugesetzt und ihr Überschuss nach Reaktionsende durch vorsichtiges Entspannen entfernt. Dann wird weiter aufgearbeitet wie beschrieben.

Feste Produkte können gegebenenfalls z.B. durch Umkristallisieren oder Umfällen weiter gereinigt werden.

Es kann von Vorteil sein, die Reaktion dosierkontrolliert durchzuführen, indem man im Lauf der Reaktion die Verbindung der Formel (II) bei Reaktionstemperatur zudosiert.

Es kann weiterhin von Vorteil sein, in Schritt A) Radikalinhibitoren, wie z.B. 2,6-Di-tert.butylphenol zuzusetzen um radikalische Nebenreaktionen weitestgehend zu vermeiden.

Alternativ kann auch der Palladiumkatalysator erst im Verlauf der Reaktion zugegeben werden oder durch Zugabe von Ligand oder Palladiumverbindung im Lauf der Reaktion erzeugt werden. Auch die simultane Zudosierung von Verbindungen der Formel (II) und Palladiumkatalysator bzw. Ligand bzw. Palladiumverbindung ist möglich.

Es ist von Vorteil, bei der Aufarbeitung eine schwach saure wässrige Lösung zu verwenden, um gegebenenfalls verbleibende Base als Salz zu binden. Die Base kann beispielsweise durch Alkalisieren und Extrahieren der Waschflüssigkeit mit einem organischen Lösungsmittel zurückgewonnen werden.

Gegebenenfalls können in einem Schritt B) die Verbindungen der Formel (III) durch Eliminierung in Verbindungen der Formel (IV) überführt werden.

Vorzugsweise wird die Eliminierung in einem organischen Lösungsmittel in Gegenwart von Base durchgeführt.

Als Basen können beispielsweise eingesetzt werden:

Erdalkali- oder Alkalimetallhydride, -hydroxide, -amide, -alkoholate wie beispielsweise Natriumhydrid, Natriumamid, Lithium-diethylamid, Natriummethylat, Natriumethylat, Kaliumtert.-butylat, Natriumhydroxid, Kaliumhydroxid, tertiäre Amine, wie Trimethylamin, Triethylamin, Tributylamin, Di-isopropyl-ethylamin, Piperidin, N-Methylpiperidin, N,N-Dimethylaminopyridin, Diazabicyclooctan (DABCO), Diazabicyclononen (DBN) oder Diazabicycloundecen (DBU) sowie N-heteroaromatische Verbindungen wie beispielsweise Pyridin und 3-N,N-Dimethylaminopyridin.

Bei Verbindungen der Formel (III), in denen Y für Chlor steht, werden in Abhängigkeit vom Substitutionsmuster vorzugsweise lithiumorganische Verbindungen wie Methyl- oder n-Butyllithium oder Alkali- oder Erdalkalimetall-hydride, -amide oder -alkoholate eingesetzt. Dabei haben sich beispielsweise Kalium-tert.-butanolat, Natrium oder Natriumamid in Ammoniak oder verschiedenen Ethem, sowie Methyllithium oder n-Butyllithium in THF oder Diethylether besonders bewährt.

Bei Verbindungen der Formel (III), in denen Y für Brom steht, werden vorzugsweise neben den für die analogen Chlorverbindungen genannten Basen und Base/Lösungsmittelkombinationen ebenfalls bevorzugt Hydroxide in polaren Lösungsmitteln eingesetzt.

Die Eliminierung kann beispielsweise bei Temperaturen von -20 bis 200°C durchgeführt werden, bevorzugt bei 20 bis 180°C, besonders bevorzugt bei 80 bis 180°C.

Die Reaktionsdauer in Schritt B) kann beispielsweise 0,5 bis 72 Stunden, bevorzugt 2 bis 24 Stunden betragen.

Der Druck in Schritt B) ist unkritisch und kann beispielsweise 0,5 bis 100 bar, bevorzugt 0,8 bis 3 bar betragen. Besonders bevorzugt ist Umgebungsdruck.

Die Aufarbeitung in Schritt B) kann in an sich bekannter Weise beispielsweise durch Extraktion und anschließendes Entfernen flüchtiger Bestandteile erfolgen.

In einer Ausführungsform des erfindungsgemäßen Verfahrens kann Schritt B) beispielsweise auch so durchgeführt werden, dass der Reaktionsmischung, die gemäß Schritt A) erhalten wird ohne Zwischenisolierung von Verbindungen der Formel (III) Base zugesetzt wird.

Das erfindungsgemäße Verfahren eignet sich insbesondere zur Herstellung von 4-Ethinyl-1,5-dimethyl-3-(trifluoromethyl)-1H pyrazol, 4-(2-Chlorethenyl)-1,5-dimethyl-3-(trifluoromethyl)-1*H*-pyrazol, 4-(2-Bromethenyl)-1,5-dimethyl-3-(trifluoromethyl)-1*H* pyrazol, 4-(2-Methoxycarbonylethenyl)-1,5-dimethyl-3-(trifluoromethyl)-1*H*-pyrazol und 4-(2-Ethoxycarbonylethenyl)-1,5-dimethyl-3-(trifluoromethyl)-1*H*-pyrazol, die von der Erfindung als Substanzen ebenfalls umfasst sind.

Die erfindungsgemäß herstellbaren Verbindungen der Formel (III) und (IV) eignen sich insbesondere zur Anwendung in einem Verfahren zur Herstellung von Arzneimitteln und Agrochemikalien.

Die erfindungsgemäß herstellbaren Verbindungen der Formeln (III) und (IV) und die zuletzt genannten Einzelverbindungen der Formel (IV) eignen sich vorzugsweise zur Herstellung von Akariziden.

Ein Vorteil der Erfindung liegt darin, dass erfindungsgemäß Arylvinylhalogenide und -sulfonaten und Arylalkine mit hohen Ausbeuten in einfacher und effizienter gewonnnen werden können.

## Beispiele

### Beispiel 1:

### Herstellung von 4-(2-Chlorethenyl)-1,5-dimethyl-3-(trifluoromethyl)-1*H*-pyrazol

2,0 g 4-Brom-1,5-dimethyl-3-(trifluoromethyl)-1H-pyrazol werden mit 18,5 mg Palladiumacetat, 73,2 mg Di (tert.-butyl)phenylphosphan, 60,3 mg Tetrabutylammoniumbromid, 10 mg Hydrochinon, 1,8 g Dicyclohexylmethylamin und 23 ml Dimethylacetamid in einem Edelstahlautoklaven vorgelegt. In diesen Autoklaven werden bei tiefer Temperatur 2,6 g Vinylchlorid einkondensiert und anschließend der Autoklav verschlossen. Anschließend wird für 20 h auf 140°C erhitzt. Dann wird abgekühlt, entspannt, verwässert und extraktiv aufgearbeitet. Es werden 1,53 g (83% d. Th.) 4-(2-Chlorethenyl)-1,5-dimethyl-3-(trifluoromethyl)-1H-pyrazol isoliert.

### Beispiel 2

### Herstellung von 4-Ethinyl-1,5-dimethyl-3-(trifluoromethyl)-1*H* pyrazol

1,27 g (5,67 mmol) 4-(2-Chlorethenyl)-1,5-dimethyl-3-(trifluoromethyl)-1H-pyrazol aus Beispiel 1 werden in 40 ml trockenen THF gelöst und unter Schutzgas-Atmossphäre gesetzt. Nach Abkühlen der Reaktionslösung auf 0°C trägt man 1.91 (17.0 mmol) Kalium-tertiät-Butanolat ein und rührt nach vollständiger Zugabe für 3 stunden bei 20°C. Nach Reaktionsende fügt man eine gesättigte Ammoniumchloridlösung zu (25 ml) und extrahiert zweimal mit je 30 ml Methylenchlorid. Die vereinigten organischen Phasen werden über Magnesiumsulfat getrocknet, das Lösungsmittel destillativ entfernt und 4-Ethinyl-1,5-dimethyl-3-(trifluoromethyl)-1*H*-pyrazol mit einer Ausbeute von 79 % d.Th. als mikrokristalliner Feststoff isoliert.

**Patentansprüche**

1. Verfahren zur Herstellung von Arylvinylhalogeniden oder -sulfonaten sowie Arylalkinen, **dadurch gekennzeichnet, dass** in einem Schritt

A)

- Verbindungen der Formel (I),

$$Ar\text{-}[X]_n \,, \qquad (I)$$

in der

n für eins oder zwei steht und

Ar für einen substituierten oder unsubstituierten aromatischen oder für einen substituierten oder unsubstituierten polyaromatischen Rest und

X jeweils unabhängig für Chlor, Brom, Iod, ein Sulfonat oder ein Diazoniumsalz steht,

- in Gegenwart von Palladium-Katalysator und
- in Gegenwart mindestens einer Base
- mit Verbindungen der Formel (II),

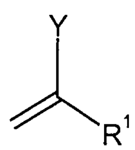

(II)

in der

Y für Fluor, Chlor, Brom, Iod, ($C_1$-$C_{12}$-Alkyl)sulfonyloxy oder ($C_1$-$C_{12}$-Halogenalkyl)sulfonyloxy und

$R^1$ für Wasserstoff, Cyano, $C_1$-$C_{12}$-Alkyl, $C_5$-$C_{14}$-Aryl, Fluor, Chlor, COO($C_5$-$C_{14}$-Aryl), COO ($C_1$-$C_{12}$-Alkyl), CON($C_5$-$C_{14}$-Aryl)$_2$ CON($C_1$-$C_{12}$-Alkyl)$_2$, OCO($C_5$-$C_{14}$-Aryl) oder OCO($C_1$-$C_{12}$-Alkyl) steht. zu Verbindungen der Formel (III) umgesetzt werden,

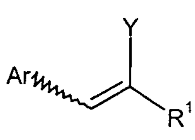

(III)

und gegebenenfalls in einem Schritt

B) die Verbindungen der Formel (III) durch Umsetzung mit Base zu Verbindungen der Formel (IV)

$$Ar-\equiv-R^1 \qquad (IV)$$

umgesetzt werden.

2. Verfahren nach Anspruch 1, **dadurch gekennzeichnet, dass** Schritt A) in Gegenwart eines Salzes durchgeführt wird.

3. Verfahren nach mindestens einem der Ansprüche 1 und 2, **dadurch gekennzeichnet, dass** Schritt A) in Gegenwart von Lösungsmittel durchgeführt wird.

4. Verfahren nach mindestens einem der Ansprüche 1 bis 3, **dadurch gekennzeichnet, dass** Ar für carbocyclische aromatische Reste mit 6 bis 24 Gerüstkohlenstoffatomen, für carbocyclische bis- oder trisaromatische Reste mit 6 bis 10 Kohlenstoffatomen pro aromatischen Rest oder für heteroaromatische Reste mit 5 bis 24 Gerüstatomen sthet, in denen keines, ein, zwei oder drei Gerüstatome pro Cyclus, im gesamten Molekül mindestens jedoch ein Gerüstatom Heteroatome sind, die ausgewählt sind aus der Gruppe Stickstoff, Schwefel oder Sauerstoff und wobei die carbocyclischen aromatischen Reste, die carbocyclischen bis- oder trisaromatischen Reste oder die heteroaromatischen Reste weiterhin mit bis zu fünf gleichen oder verschiedenen Substituenten pro Cyclus substituiert sein können, die ausgewählt sind aus der Gruppe Hydroxy, Chlor, Fluor, Nitro, Cyano, freies oder geschütztes Formyl, $C_1$-$C_{12}$-Alkyl, $C_5$-$C_{14}$-Aryl, $C_6$-$C_{15}$-Arylalkyl, -PO-[($C_1C_8$)-Alkyl]$_2$, -PO-[($C_5$-$C_{14}$)-Aryl]$_2$, -PO-[($C_1$-$C_8$)-Alkyl) ($C_5$-$C_{14}$)-Aryl)], Tri($C_1$-$C_8$alkyl)siloxyl oder aus Resten der Formel (Va-f),

$$A\text{-}B\text{-}D\text{-}E \qquad (Va)$$

$$A\text{-}E \qquad (Vb)$$

$$A\text{-}SO_2\text{-}E \qquad (Vc)$$

$$A\text{-}B\text{-}SO_2R^3 \qquad (Vd)$$

$$A\text{-}SO_3W \qquad (Ve)$$

$$A\text{-}COW \qquad (Vf)$$

in denen unabhängig voneinander

A fehlt oder für einen $C_1$-$C_8$-Alkylenrest steht und

B fehlt oder für Sauerstoff, Schwefel oder $NR^2$ steht, wobei $R^2$ Wasserstoff, $C_1$-$C_8$-Alkyl, $C_6$-$C_{15}$-Arylalkyl oder $C_5$-$C_{14}$-Aryl bedeutet und

D für eine Carbonyl-Gruppe steht und

E für $R^3$, $OR^3$, $NHR^4$ oder $N(R^4)_2$ steht, wobei $R^3$ für $C_1$-$C_8$-Alkyl, $C_6$-$C_{15}$-Arylalkyl, $C_1$-$C_8$-Halogenalkyl oder $C_5$-$C_{14}$-Aryl und

$R^4$ jeweils unabhängig für $C_1$-$C_8$-Alkyl, $C_6$-$C_{15}$-Arylalkyl oder $C_6$-$C_{14}$-Aryl oder $N(R^4)_2$ zusammen für einen cyclischen Aminorest steht und

W für OH, $NH_2$, oder OM steht, wobei M ein Alkalimetallion, ein halbes Äquivalent eines Erdalkalimetallions,

ein Ammoniumion oder ein organisches Ammoniumion bedeuten kann.

5. Verfahren nach mindestens einem der Ansprüche 1 bis 4, **dadurch gekennzeichnet, dass** X für Chlor, Brom, Iod, Trifluormethansulfonyloxy oder Nonafluorbutansulfonyloxy steht.

6. Verfahren nach mindestens einem der Ansprüche 1 bis 5, **dadurch gekennzeichnet, dass** Y für Fluor, Chlor, Brom, Iod, Methansulfonyloxy, p-Tolylsulfonyloxy, Trifluormethansulfonyloxy oder Nonafluorbutansulfonyloxy steht.

7. Verfahren nach mindestens einem der Ansprüche 1 bis 6, **dadurch gekennzeichnet, dass** R' für Wasserstoff, Cyano, $C_1$-$C_{12}$-Alkyl, $C_5$-$C_{14}$-Aryl, Fluor, Chlor, COO($C_5$-$C_{14}$-Aryl), COO($C_1$-$C_{12}$-Alkyl), OCO($C_5$-$C_{14}$-Aryl) oder OCO($C_1$-$C_{12}$-Alkyl) steht.

8. Verfahren nach mindestens einem der Ansprüche 1 bis 7, **dadurch gekennzeichnet, dass** als Verbindungen der Formel (II) Vinylfluorid, Vinylchlorid, Vinylbromid, 2-Chloracrylnitril, 2-Chloracrylsäuremethylester, 2-Chloracrylsäurebutylester, 1,1-Dichlorethylen, 1,1-Difluorethylen und 4-(1-Chlorethenyl)-1,5-dimethyl-3-(trifluoromethyl)-1H-pyrazol eingesetzt werden.

9. Verfahren nach mindestens einem der Ansprüche 1 bis 8, **dadurch gekennzeichnet, dass** als Palladiumkatalysator Palladiumkomplexe eingesetzt werden, die aus geeigneten Liganden in der Reaktionslösung erzeugt werden, oder in Form bereits isolierter Palladium-Komplexe eingesetzt werden.

10. Verfahren nach mindestens einem der Ansprüche 1 bis 9, **dadurch gekennzeichnet, dass** als Basen ionische Basen, Amine oder N-heteroaromatische Verbindungen eingesetzt werden.

11. Verfahren nach mindestens einem der Ansprüche 1 bis 10, **dadurch gekennzeichnet, dass** Schritt A) dosierkontrolliert durchgeführt wird.

12. Verfahren nach mindestens einem der Ansprüche 1 bis 11, **dadurch gekennzeichnet, dass** in Schritt A) Radikalinhibitoren zugesetzt werden.

13. Verfahren nach mindestens einem der Ansprüche 1 bis 12, **dadurch gekennzeichnet, dass** als Basen Erdalkali- oder Alkalimetallhydride, -hydroxide, -amide, -alkoholate, tertiäre Amine und/oder N-heteroaromatische Verbindungen eingesetzt werden.

14. Verfahren nach mindestens einem der Ansprüche 1 bis 13, **dadurch gekennzeichnet, dass** 4-Ethinyl-1,5-dimethyl-3-(trifluoromethyl)-1*H*-pyrazol,4-(2-Chlorethenyl)-1,5-dimethyl-3-(trifluoromethyl)-1*H*-pyrazol,4-(2-Bromethenyl)-1,5-dimethyl-3-(trifluoromethyl)-1*H*-pyrazol, 4-(2-Methoxycarbonylethenyl)-1,5-dimethyl-3-(trifluoromethyl)-1H-pyrazol oder 4-(2-Ethoxycarbonylethenyl)-1,5-dimethyl-3-(trifluoromethyl)-1H-pyrazol hergestellt wird.

15. Verwendung von Verbindungen, die nach einem oder mehreren der Ansprüche 1 bis 14 hergestellt wurden, in einem Verfahren zur Herstellung von Arzneimitteln und Agrochemikalien.

16. Verwendung von Verbindungen, die nach Anspruch 14 hergestellt wurden, in einem Verfahren zur Herstellung von Akariziden.

17. 4-Ethinyl-1 ,5-dimethyl-3-(trifluoromethyl)-1*H*-pyrazol, 4-(2-Chlorethenyl)-1,5-dimethyl-3-(trifluoromethyl)-1H-pyrazol, 4-(2-Bromethenyl)-1,5-dimethyl-3-(trifluoromethyl)-1Hpyrazol, 4-(2-Methoxycarbonylethenyl)-1,5-dimethyl-3-(trifluoromethyl)-1*H*-pyrazol und 4-(2-Ethoxycarbonylethenyl)-1,5-dimethyl)-3-(trifluoromethyl)-1*H*-pyrazol